# EUROPEAN PATENT SPECIFICATION

(11) **EP 1 506 963 B1**
(45) Date of publication and mention of the grant of the patent: **13.04.2005**
(21) Application number: 03425693.3
(22) Date of filing: 28.10.2003
(51) Int. Cl.: C07D 307/87, C07C 255/50, C07F 3/02

(54) **Method for the preparation of citalopram**
Verfahren zur Herstellung von Citalopram
Procédé de préparation de Citalopram

(43) Date of publication of application: 16.02.2005
(73) Proprietor: Adorkem Technology SpA, 24062 Costa Volpino (Bergamo) (IT)
(72) Inventor: Cotticelli, Giovanni, 20063 Cernusco sul Naviglio (IT); di Lernia, Gianluca, 20139 Milano (IT); Silvia, Milanesi, 27042 Bressana Bottarone Pavia (IT)
(74) Representative: Pistolesi, Roberto

(56) References cited:
- EP-A- 0 171 943
- WO-A-98/19512
- WO-A-02/060886
- WO-A-03/029236
- US-B1- 6 229 026

## Description

The present invention relates to a process for preparation, in just one series of reactions, of 1-[3-(dimethylamino)propyl]-1-(4-fluorophenyl)-1,3-dihydro-5-isobenzofurancarbonitrile and its pharmaceutically acceptable salts.

### BACKGROUND OF THE INVENTION

The mentioned compound, whose formula of structure is here-below reported, 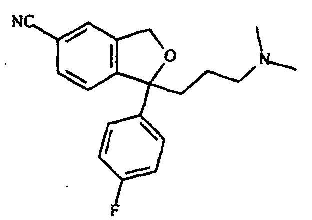 is a well known active drug, better known under its International Denomination "citalopram", which is used in bromhydrate form for the preparation of pharmaceutical compositions for the treatment of depression.

Citalopram was first disclosed in the Belgian patent application BE-850401 (and in its equivalent US patent US- 4,136,193); several patent documents also describe methods for its preparation.

Preferably, EP-171943 describes a synthetic method with two consecutive Grignard reactions starting from 5-cyanophthalide, the first one with 4-fluorophenylmagnesium bromide, and the second one from the thus-obtained magnesium derivate (formula A), 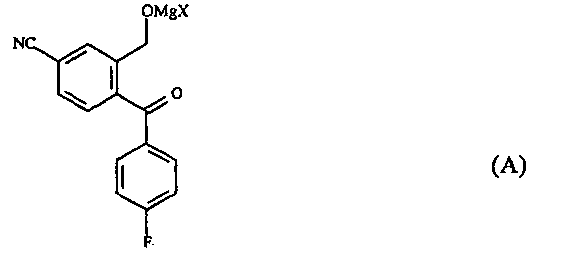 with 3-(dimethylamino)propylmagnesium chloride to obtain first a magnesium intermediate (formula B) 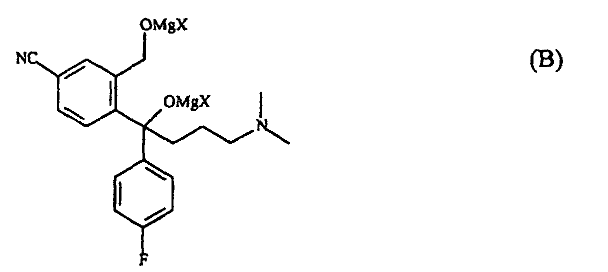 and then, after an acid hydrolysis, a diol (formula C) precursor of citalopram.

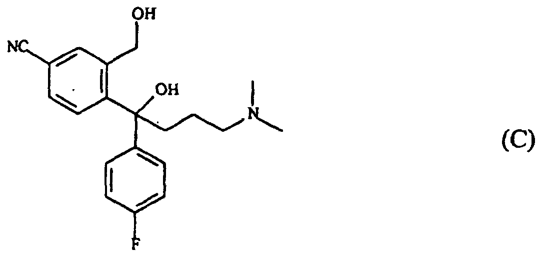

In particular, EP-171943 describes the reaction between 4-fluorophenylmagnesium bromide and 5-cyanophthalide as a slow mechanism so as to state that the intermediate of formula (A) is obtained after a slow transformation from another intermediate (A') as reported here-below:

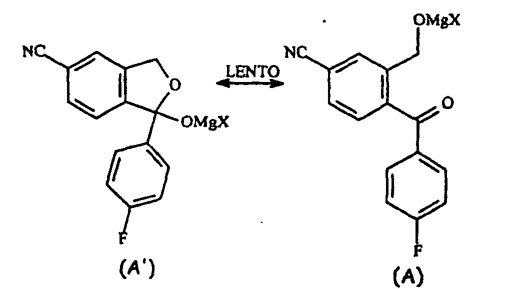

The subsequent reaction between intermediate (A) and 3-(dimethylamino)propylmagnesium chloride is described as slow too (the examples disclose a reaction time of one night).

### SUMMARY OF THE INVENTION

The present invention describes a preparation of citalopram from 5-cyanophthalide by the following route: 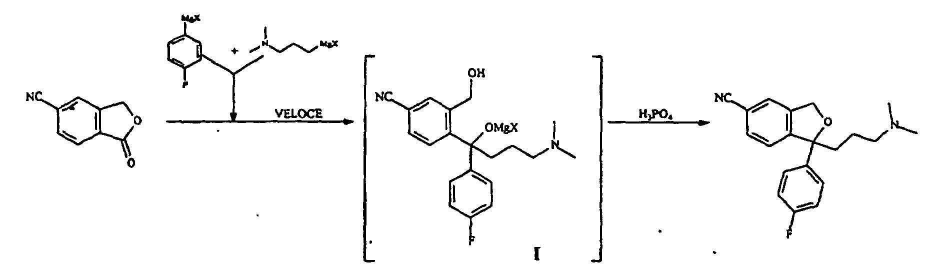 where X is an halogen, preferably chlorine or bromine.

This process is very easy and fast, it doesn't involve the formation of the above-mentioned intermediates (A),(A') and (C) and it allows to obtain citalopram without the isolation of intermediate I.

More in details, intermediate I is synthesized from 5-cyanophthalide by adding a mixture of 4-fluorophenyl magnesium bromide and 3-dimethylaminopropyl magnesium chloride. This reaction, contrarily to EP-171943, is basically instantaneous; besides, at the end of the mixture addition, cyclization is possible by directly adding an acid (preferably orto phosphoric acid 85%) to the reaction mixture, with great operative advantages; after solvent distillation (preferably tetrahydrofuran) the reaction is finished in about two hours. The subsequent isolation of citalopram is carried out with well known extraction methods.

The present invention relates to a novel method for the preparation of citalopram which comprises:
a) the reaction of 5-cyanophthalide with a mixture of 4-fluorophenyl magnesium halogenide and 3-dimethylaminopropyl magnesium halogenide;
b) the resulting mixture is treated with an organic or inorganic acid having a pK comprised between 0 and 3, preferably between 2 and 3,

In a preferred embodiment of the invention, 4-fluorophenyl magnesium bromide and 3-dimethylaminopropyl magnesium chloride are employed; furthermore, said acid having a pK comprised between 0 and 3 is preferably orto phosphoric acid.

According to a preferred way to proceed, the process according to the invention is carried out "one pot", without isolating the intermediate products until obtainment of citalopram.

The reaction is preferably carried out in an organic polar aprotic solvent, preferably tetrahydrofuran and/or toluene. In practice, the Grignard solution is prepared by adding a solution of 4-fluorobromobenzene in said organic polar aprotic solvent (preferably tetrahydrofuran and/or toluene) to magnesium turnings in presence of traces of iodide at solvent reflux temperature (at 50ö70°C, preferably 70°C for tetrahydrofuran) and cooling to room temperature, after about 30 minutes. Separately a solution of ethylbromide in the same organic solvent is added to magnesium turnings in presence of traces of iodide at solvent reflux temperature; maintaining the mixture at this temperature, 3-dimethylaminopropyl chloride is added to the same solvent, reflux is maintained for further thirty minutes, then it's cooled to room temperature. The two solutions are then mixed together at room temperature.

The mixture is added to a 5-cyanophthalide suspension in the same organic polar aprotic solvent, preferably tetrahydrofuran, at -20ö+20°C, preferably at -10ö0°C. The reaction is usually finished at the end of the addition.

The acid having a pK comprised between 0 and 3, preferably orto-phosphoric acid, is then added to the reaction mixture at -10ö+20°C, preferably at 0ö+10°C; the mixture is then heated to 55ö85°C, preferably to about 65°C, in order to distil all the tetrahydrofuran. At the end of the distillation, the mixture is kept at 60ö90°C, preferably at 70ö80°C for 1ö3 hours, preferably for about two hours, to give citalopram.

In a preferred embodiment of the reaction, from 1.8 to 2.0 moles of 4-fluorophenyl magnesium halogenide are used, preferably about 1.8 moles, and from 1.09 to 1.2 moles of 3-dimethylaminopropyl magnesium halogenide, preferably about 1.1 moles, are used for each mole of 5-cyanophthalide.

According to the best mode to carry out the invention, in order to reduce the production of possible undesired by-products, from 1.7 to 1.6 moles of 4-fluorophenyl magnesium halide, preferably about 1.64, are used for each mole of 3-dimethylaminopropyl magnesium halide; this molar ratio can be for example obtained by mixing 3.4 parts by weight of a 20% 4-fluorophenylmagnesium halide solution in said organic solvent and 1 part by weight of a 30% dimethylaminopropylmagnesium chloride solution in said organic solvent.

Moreover, the reaction is carried out in from 1.0 to 1.6 litres of solvent, preferably about 1.2 litres, for each mole of 5-cyanophthalide.

The acid having a pK comprised between 0 and 3, and in particular the orto phosphoric acid, is normally used at a concentration between 55 and 95% by weight, a concentration of about 85% being particularly preferred.

The citalopram is obtained by extraction preferably with toluene/water firstly in an acid environnement and then in a basic environnement.

On the whole, the invention allows to reduce considerably the reaction and the work-up times, according to a novel and simple way of synthesis. The following examples are for illustraton only and are not intended to limit the invention.

### EXAMPLE

### 1) 20% solution of 4-fluorophenylmagnesium bromide in tetrahydrofuran

53.5 g of magnesium turnings (2.2 mol) and 0.3 g of iodide are charged into a 4-litres reactor at room temperature under nitrogen. The mixture is then heated to 70°C and a solution of 369.5 g (2.11 mol) of 4-fluorobromobenzene in 1960 ml tetrahydrofuran (5.3 volumes on 4-fluorobromobenzene) is added drop wise, in one hour. After addition the mixture is heated to reflux temperature (68ö70°C) for 30 minutes, then it's cooled to 25°C.

2000g of a 20% solution of 4-fluorophenylmagnesium bromide in tetrahydrofuran are obtained (to be kept under nitrogen and protected from the light).

### 2) 30% solution of dimethylaminopropyl magnesium chloride in tetrahydrofuran

39.22 g (1.61 mol) of magnesium turnings e 0.3 g of iodide are charged into a 2-liters reactor at room temperature under nitrogen: The mixture is then heated to 70°C and a solution of 4.53 ml (0.061 mol) of ethylbromide in 72 ml of tetrahydrofuran is added drop wise in 15 minutes. The reaction is quickly seeded. A solution of 208.77 g (1.90 mol) of dimethylaminopropyl chloride in 545 ml of tetrahydrofuran is added drop wise. After addition the mixture is heated to reflux temperature (68ö70°C) for 30 minutes, then it's cooled to 25°C.

774.1 g of a 30% of dimethylaminopropylmagnesium chloride solution in tetrahydrofuran are obtained (to be kept under nitrogen and protected from the light).

### 3) Citalopram

A mixture of 1150 g (1.15 mol) of a 20% 4-fluorophenylmagnesium bromide solution and 338 g (0.69 mol) of a 30% dimethylaminopropylmagnesium chloride solution is prepared at room temperature.

The resulting mixture is then added in about 2 hours to a mixture of 100 g (0.63 moles) of 5-cyanophthalide in 750 ml of tetrahydrofuran, under nitrogen at -0ö0°C. After addition (see note 1), 550 ml of orto-phosphoric acid 85% are added drop wise, keeping the temperature below 10°C. After addition the mixture is heated to 66°C and tetrahydrofuran is distilled; the mixture is then heated to 70ö80°C for 2 hours. The reaction is finished from HPLC control. 1100 ml of water and 650 ml of toluen are added.

The phases are separated; the aqueous phase is extracted with 200 ml of toluene to give a new aqueous phase.

9 g of active carbon are added to this new aqueous phase; the mixture is stirred for one hour at 25°C, then it's filtered on a supra panel and washed with water (3x50ml). The filtered is cooled to 5-10°C, 650 ml of toluene are added and about 1300 ml of ammonium hydroxide solution 30% are added till pH 9.5 (keeping the temperature below 15°C). The possible undissolved salts are filtered washing the panel with toluene (about 200 ml) and the phases are separated. 300 ml of water are added to the organic phase. The phases are separated. The organic phase is evaporated to crude citalopram as an oil. Crude Citalopram: 177 g

### 4) Citalopram bromhydrate (salyfication)

177 g of crude citalopram obtained from the previous step are dissolved in 300 ml of acetone; it is cooled to 0ö5°C and about 30 ml of hydrobromic acid 62% are added till pH 1. The suspension is stirred at 0ö5°C for one hour and the solvent is then evaporated under vacuum. 200 ml of acetone are added and the solvent is evaporated under vacuum at 40°C; 250 ml of acetone are added and the suspension is stirred at 0ö5°C for a night. The panel is washed with 3x50 ml of acetone at 0ö5°C. The crude citalopram bromhydrate is dried under vacuum at 60°C.
Crude Citalopram bromhydrate: 95,1g

### 5) Citalopram bromhydrate (purification)

95 g of crude citalopram bromhydrate are suspended in 192 ml of deionised water, and the suspension is heated to 60°C to give a solution. 5.7 g of active carbon are added and the mixture is stirred 30 minutes at 60°C. It's filtered on a supra panel at 60°C and the panel is washed with deionised water at 60°C (2x25 ml).

The filtered solution is charged in a 500 ml reactor, it's cooled to 0°C and it's stirred for a night. On the following day 140 ml of deionised water are added at 0°C and it's stirred for 5 hours. It's filtered washing the panel with 70 ml of deionised cold water.

The pure citalopram bromhydrate is dried under vacuum at 60°C.
Pure citalopram bromhydrate: 68.8g (molar yield: 27%; w/w yield: 68.8%).

NOTE 1: At the end of the addition of the mixture of the two Grignards to the suspension of 5-cyanophthalide, an NMR analysis (BRUKER AMX 3-400) is carried out on a sample.

| ¹H-NMR (DMSO-d6) | | | |
|---|---|---|---|
| δ(ppm) | Multiplicity | N° H | Attribution |
| 7.83-7.70 | m | 3 | H4, H5, H6 |
| 7.22-7.05 | m | 4 | H7, H8 |
| 5.1 | s | 1 | H2 |
| 4.52-4.48 | d | 1 | H1 |
| 4.00-3.96 | d | 1 | H1' |
| 2.98-2.16 | m | 6 | H9, H10, H11 |
| 1.69 | s | 6 | H12 |

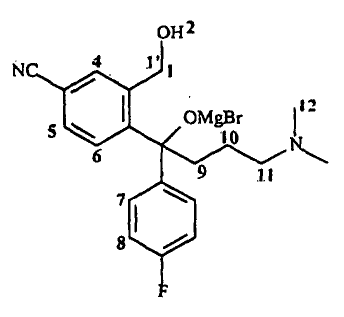

This ¹H-NMR is compared to the compound having 2 free hydroxy groups described in EP-171943, here-below reported. By comparison, it's showed that the peak at 6.50 ppm, which corresponds to the hydroxy group in position 3, is lacking in intermediate I.

| ¹H-NMR (DMSO-d6) | | | |
|---|---|---|---|
| δ(ppm) | Multiplicity | N° H | Attribution |
| 7.89-7.74 | m | 3 | H4, H5, H6 |
| 7.27-7.07 | m | 4 | H7, H8 |
| 6.50 | s | 1 | H3 |
| 5.16 | s | 1 | H2 |
| 4.60-4.56 | d | 1 | H1 |
| 4.08-4.04 | d | 1 | H1' |
| 2.29-2.23 | m | 2 | H9 |
| 2.16-2.11 | m | 2 | H11 |
| 2.01 | s | 6 | H12 |
| 1.39-1.19 | m | 2 | H10 |

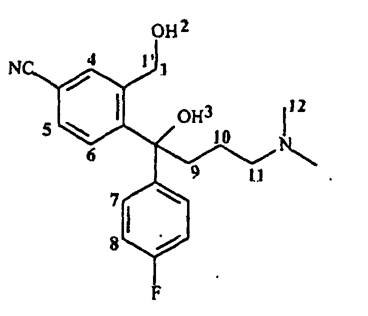

## Claims

1. A process for the preparation of citalopram **characterized in that** (a) 5-cyanophthalide is treated with a mixture of 4-fluorophenyl magnesium halide and 3-dimethylaminopropyl magnesium halide and (b) the obtained mixture is treated with an organic or inorganic acid.

2. A process according to claim 1, **characterized by** the use of from 1.8 to 2.0 moles of 4-fluorophenyl magnesium halide, preferably about 1.8, for each mole of 5-cyanophthalide.

3. A process according to claim 1, **characterized by** the use of from 1.09 to 1.2 moles of 3-dimethylaminopropyl magnesium halide, preferably about 1.1, for each mole of 5-cyanophthalide.

4. A process according to claim 1, **characterized by** the fact that from 1.7 to 1.6 moles of 4-fluorophenyl magnesium halide, preferably about 1.64, are used for each mole of 3-dimethylaminopropyl magnesium halide.

5. A process according to claims 1-4, **characterized by** the fact that 4-fluorophenyl magnesium halide is a bromide.

6. A process according to claims 1-4, **characterized by** the fact that 3-dimethylaminopropyl magnesium halide is a chloride.

7. A process according to any one of the previous claims, **characterized by** the fact that said acid has a pK comprised from 0 to 3, preferably from 2 to 3.

8. A process according to claim 7, **characterized by** the fact that said acid is orto-phosphoric acid.

9. A process according to claims 7-8, **characterized by** the fact that the acid is used in a concentration comprised from 55 to 95% by weight, preferably in concentration of about 85% by weight.

10. A process according to any one of the previous claims, **characterized by** the fact that the process is carried out in an organic polar aprotic solvent.

11. A process according to claim 10, **characterized by** the fact that the process is carried out in from 1.0 to 1.6 litres of solvent, preferably in about 1.2 litres, for each mole of 5-cyanophthalide.

12. A process according to claims 10-11, **characterized by** the fact that the solvent is selected from tetrahydrofuran and/or toluene.

13. A process according to any one of previous claims, **characterized by** the fact that the step (a) is carried out at -20ö+20°C, preferably at -10ö0°C.

14. A process according to any one of previous claims, **characterized by** the fact that the step (b) is carried out at -10ö+20°C, preferably at 0ö+10°C.

15. A process according to any one of previous claims, **characterized by** the fact of being carried out without isolating the intermediate products.

16. Compound of formula: 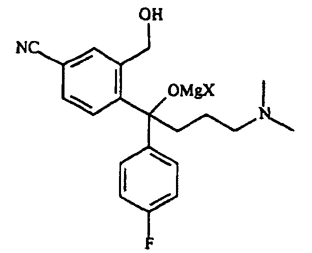 where X is an halogen, preferably chlorine or bromine.

17. Use of a compound according to claim 16 as an intermediate in the preparation of citalopram.

## Patentansprüche

1. Verfahren zur Herstellung von Citalopram, **dadurch gekennzeichnet, dass** (a) 5-Cyanophthalid mit einer Mischung von 4-Fluorphenylmagnesiumhalogenid und 3-Dimethylaminopropylmagnesiumhalogenid behandelt wird und (b) die erhaltene Mischung mit einer organischen oder anorganischen Säure behandelt wird.

2. Verfahren nach Anspruch 1, **gekennzeichnet durch** die Verwendung von 1,8 bis 2,0 Mol 4-Fluorphenylmagnesiumhalogenid, bevorzugt etwa 1,8, für jedes Mol 5-Cyanophthalid.

3. Verfahren nach Anspruch 1, **gekennzeichnet durch** die Verwendung von 1,09 bis 1,2 Mol 3-Dimethylaminopropylmagnesiumhalogenid, bevorzugt etwa 1,1, für jedes Mol 5-Cyanophthalid.

4. Verfahren nach Anspruch 1, **gekennzeichnet durch** die Tatsache, dass 1,7 bis 1,6 Mol 4-Fluorphenylmagnesiumhalogenid, bevorzugt etwa 1,64, für jedes Mol 3-Dimethylaminopropylmagnesiumhalogenid verwendet werden.

5. Verfahren nach den Ansprüchen 1 bis 4, **gekennzeichnet durch** die Tatsache, dass das 4-Fluorphenylmagnesiumhalogenid ein Bromid ist.

6. Verfahren nach den Ansprüchen 1 bis 4, **gekennzeichnet durch** die Tatsache, dass das 3-Dimethylaminopropylmagnesiumhalogenid ein Chlorid ist.

7. Verfahren nach irgendeinem der vorhergehenden Ansprüche, **gekennzeichnet durch** die Tatsache, dass die Säure einen pK von 0 bis 3, bevorzugt von 2 bis 3, aufweist.

8. Verfahren nach Anspruch 7, **gekennzeichnet durch** die Tatsache, dass die Säure Orthophosphorsäure ist.

9. Verfahren nach den Ansprüchen 7-8, **gekennzeichnet durch** die Tatsache, dass die Säure in einer Konzentration von 55 bis 95 Gew.-%, bevorzugt in einer Konzentration von etwa 85 Gew.-%, verwendet wird.

10. Verfahren nach irgendeinem der vorhergehenden Ansprüche, **gekennzeichnet durch** die Tatsache, dass das Verfahren in einem organischen, polaren, aprotischen Lösungsmittel durchgeführt wird.

11. Verfahren nach Anspruch 10, **gekennzeichnet durch** die Tatsache, dass das Verfahren in 1,0 bis 1,6 I Lösungsmittel, bevorzugt etwa 1,2 I, für jedes Mol 5-Cyanophthalid durchgeführt wird.

12. Verfahren nach den Ansprüchen 10-11, **gekennzeichnet durch** die Tatsache, dass das Lösungsmittel aus Tetrahydrofuran und/oder Toluol ausgewählt wird.

13. Verfahren nach irgendeinem der vorhergehenden Ansprüche, **gekennzeichnet durch** die Tatsache, dass der Schritt (a) bei -20 bis +20°C, bevorzugt bei -10 bis 0°C, durchgeführt wird.

14. Verfahren nach irgendeinem der vorhergehenden Ansprüche, **gekennzeichnet durch** die Tatsache, dass der Schritt (b) bei -10 bis +20°C, bevorzugt bei 0 bis +10°C, durchgeführt wird.

15. Verfahren nach irgendeinem der vorhergehenden Ansprüche, **gekennzeichnet durch** die Tatsache, dass es ohne Isolierung der Zwischenprodukte durchgeführt wird.

16. Verbindung der Formel: 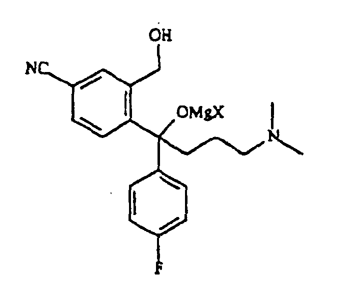 worin X ein Halogen, bevorzugt Chlor oder Brom, ist.

17. Verwendung einer Verbindung nach Anspruch 16 als ein Zwischenprodukt bei der Herstellung von Citalopram.

## Revendications

1. Procédé de préparation du citalopram **caractérisé en ce que** (a) le 5-cyanophtalide est traité par un mélange d'halogénure de 4-fluorophénylmagnésium et d'halogénure de 3-diméthylaminopropylmagnésium et (b) le mélange obtenu est traité avec un acide organique ou inorganique.

2. Procédé selon la revendication 1 **caractérisé en ce qu'**une quantité comprise entre 1,8 et 2,0 moles d'halogénure de 4-fluorophénylmagnésium, de préférence environ 1,8, est utilisée par mole de 5-cyanophtalide.

3. Procédé selon la revendication 1 **caractérisé en ce qu'**une quantité comprise entre 1,09 et 1,2 mole d'halogénure de 3-diméthylaminopropylmagnésium, de préférence environ 1,1, est utilisée par mole de 5-cyanophtalate.

4. Procédé selon la revendication 1 **caractérisé en ce qu'**une quantité comprise entre 1,7 et 1,6 moles d'halogénure de 4-fluorophénylmagnésium, de préférence environ 1,64, est utilisée par mole d'halogénure de 3-diméthylaminopropylmagnésium.

5. Procédé selon les revendications 1 à 4 **caractérisé en ce que** l'halogénure de 4-fluorophénylmagnésium est un bromure.

6. Procédé selon les revendications 1 à 4 **caractérisé en ce que** l'halogénure de 3-diméthylaminopropylmagnésium est un chlorure.

7. Procédé selon l'une quelconque des revendications précédentes, **caractérisé en ce que** ledit acide présente un pK compris entre 0 et 3, de préférence entre 2 et 3.

8. Procédé selon la revendication 7 **caractérisé en ce que** ledit acide est l'acide orthophosphorique.

9. Procédé selon les revendications 7-8, **caractérisé en ce que** l'acide est utilisé à une concentration comprise entre 55 et 95 % en poids, de préférence à une concentration d'environ 85 % en poids.

10. Procédé selon l'une quelconque des revendications précédentes, **caractérisé en ce qu'**il est réalisé dans un solvant aprotique polaire organique.

11. Procédé selon la revendication 10, **caractérisé en ce qu'**il est réalisé dans 1,0 à 1,6 litre de solvant, de préférence dans 1,2 litre, par mole de 5-cyanophtalide.

12. Procédé selon les revendications 10-11, **caractérisé en ce que** le solvant est le tétrahydrofuranne et/ou le toluène.

13. Procédé selon l'une quelconque des revendications précédentes, **caractérisé en ce que** l'étape (a) est réalisée à une température comprise entre -20 et +20 °C, de préférence entre -10 et 0 °C.

14. Procédé selon l'une quelconque des revendications précédentes, **caractérisé en ce que** l'étape (b) est réalisée à une température comprise entre -10 et +20 °C, de préférence entre 0 et +10°C.

15. Procédé selon l'une quelconque des revendications précédentes, **caractérisé en ce qu'**il est réalisé sans isoler les produits intermédiaires.

16. Composé de formule : 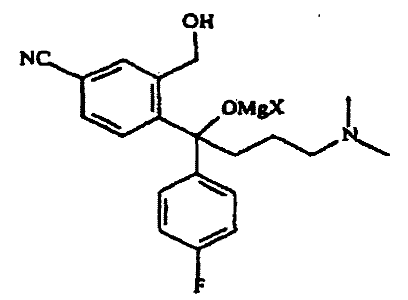 dans lequel X est un halogène, de préférence le chlore ou le brome.

17. Utilisation d'un composé selon la revendication 16 comme intermédiaire dans la préparation du citalopram.
